# EUROPEAN PATENT APPLICATION

(11) **EP 4 544 921 A1**
(43) Date of publication of application: **30.04.2025**
(21) Application number: 23827533.3
(22) Date of filing: 22.06.2023
(51) Int. Cl.: A23L 33/135, A61K 35/747, A61P 21/00, A61P 35/00, A61P 3/04, A61P 43/00

(54) **COMPOSITION COMPRISING LACTOBACILLUS GASSERI FOR PREVENTING AND TREATING SARCOPENIA**

(30) Priority: 22.06.2022 KR 20220076156; 09.02.2023 KR 20230017483
(71) Applicant: Acebiome Inc., Gangnam-gu Seoul 06164 (KR)
(72) Inventor: YOON, Yoosik, Seoul 06270 (KR); LEE, Jongkyu, Bucheon-si Gyeonggi-do 14618 (KR); YUN, Ji-Hyun, Namyangju-si Gyeonggi-do 12104 (KR)
(74) Representative: Mewburn Ellis LLP
(86) International application number: PCT/KR2023/008660
(87) International publication number: WO 2023/249423

(57) **Abstract**

The present invention relates to a composition comprising Lactobacillus gasseri for preventing and treating sarcopenia and, particularly, to: a composition comprising Lactobacillus gasseri BNR17 for preventing, treating or alleviating sarcopenia, a decrease in muscle mass and muscle function, and a decrease in muscular strength and muscular endurance, which are induced by causes such as stress, obesity, aging, drugs, tumors, and the like.

## Description

### [Technical Field]

The present invention relates to a composition for preventing or treating sarcopenia including *Lactobacillus gasseri,* and more particularly to a composition including *Lactobacillus gasseri* BNR17 for preventing, treating, or alleviating sarcopenia, a decrease in muscle mass and muscle function, and a decrease in muscular strength and muscular endurance, which are caused by factors such as stress, obesity, aging, drugs, tumors, etc.

### [Background Art]

Lactobacillus is gram-positive bacteria that mainly produce lactic acid as a result of fermentation, and is also called lactic acid bacteria. It includes bacteria belonging to family Lactobacillaceae of order Lactobacillales, and is distributed in fermented foods such as yogurt, kimchi, etc., the intestines, and milk.

Lactic acid bacteria are known to be beneficial to human health, such as preventing the proliferation of harmful bacteria in the intestines and regulating immunity to a normal range. Conventional techniques using lactic acid bacteria include Korea Patent Application Publication No. 10-2013-0002545 (Novel lactic acid bacteria and composition for preventing and treating diabetes including the same), Korean Patent Application Publication No. 10-2018-0087662 (Pharmaceutical composition for treating colorectal disease including cystatin and protein derived from lactic acid bacteria), and the like.

*Lactobacillus gasseri* is a lactic acid bacteria species of genus Lactobacillus. *Lactobacillus gasseri* is known to have effects on rheumatoid arthritis (Korean Patent Application Publication No. 10-2021-0043797), antifungal activity (Korean Patent No. 10-2063554), etc., but research into more diverse physiological effects thereof is needed.

*Lactobacillus gasseri* BNR17 is a strain belonging to *Lactobacillus gasseri* species isolated from breast milk of Korean women or a Korean woman or a Korean woman who was breast feeding her baby or Korean women who were breast feeding their babies.

Meanwhile, sarcopenia is a disease in which muscle mass, muscular strength, and muscle function are all decreased due to various factors. The factors that cause sarcopenia vary from person to person, but common factors include insufficient intake of protein including essential amino acids, lack of exercise, aging, hormonal changes, side effects of medication, and the like. In addition to diseases occurring in the muscles themselves, sarcopenia occurs secondary to cancer, diabetes, chronic diseases of the heart, lungs, and kidneys, hormonal diseases, degenerative diseases such as spinal stenosis, etc., and acute diseases such as infections, etc.

Symptoms of sarcopenia include muscle weakness, frailty , and fatigue. Muscle mass and muscular strength naturally decrease with age, but sarcopenia increases health risks and mortality by reducing physical function due to an excessive decrease in muscle mass and muscular strength, even when age and sex are taken into account. Patients with sarcopenia have reduced muscular strength and muscular endurance, are prone to osteoporosis, falls, and fractures, and have an increased risk of diabetes and cardiovascular disease due to a decrease in basal metabolic rate.

Sarcopenia is diagnosed by measuring muscle mass, muscular strength, and muscle function. Muscle mass in the human body is measured using methods such as dual energy X-ray absorptiometry, bioimpedance analysis, CT, MRI and the like. Muscular strength is measured by leg strength or grip strength, and muscle function is measured by walking speed test, 400-meter walk test, 6-minute walk test, etc.

There is currently no treatment method for sarcopenia, and the only way to delay occurrence of sarcopenia is through intake of protein containing essential amino acids, muscular strength training, and aerobic exercise.

Therefore, there is an increasing need for methods to improve health and quality of life by developing safe and effective methods of inhibiting sarcopenia for the human body and preventing, alleviating, or treating sarcopenia caused by various factors.

Against this technical background, the inventors of the present application have ascertained that *Lactobacillus gasseri* BNR17 is effective at preventing or treating sarcopenia, thus culminating in the present invention.

### [Disclosure]

It is an object of the present invention to provide a composition for preventing or treating sarcopenia including *Lactobacillus gasseri.* The present invention is intended to provide a method of preventing or treating sarcopenia including administering *Lactobacillus gasseri.* The present invention is intended to provide the use of the composition including *Lactobacillus gasseri* for the prevention or treatment of sarcopenia.

It is another object of the present invention to provide a composition for alleviating a decrease in muscle mass and muscle function including *Lactobacillus gasseri.* The present invention is intended to provide a method of alleviating of a decrease in muscle mass and muscle function including administering *Lactobacillus gasseri.* The present invention is intended to provide the use of the composition including *Lactobacillus gasseri* for the alleviation of a decrease in muscle mass and muscle function.

It is still another object of the present invention to provide a composition for improving muscular strength and muscular endurance including *Lactobacillus gasseri.* The present invention is intended to provide a method of improving in muscular strength and muscular endurance including administering *Lactobacillus gasseri.* The present invention is intended to provide the use of the composition including *Lactobacillus gasseri* for the improvement in muscular strength and muscular endurance.

It is yet another object of the present invention to provide an anticancer adjuvant including *Lactobacillus gasseri.*

In order to accomplish the above objects, the present invention provides a pharmaceutical composition for preventing or treating sarcopenia, including *Lactobacillus gasseri* BNR17 with accession number KCTC 10902BP or a culture thereof. The present invention also provides a method of preventing or treating sarcopenia, including administering *Lactobacillus gasseri* BNR17 with accession number KCTC 10902BP or a culture thereof. Furthermore, the present invention provides the use of *Lactobacillus gasseri* BNR17 with accession number KCTC 10902BP or a culture thereof for the manufacture of a pharmaceutical composition for preventing or treating sarcopenia.

The present invention provides a food composition for alleviating a decrease in muscle mass and muscle function, including *Lactobacillus gasseri* BNR17 with accession number KCTC 10902BP or a culture thereof. The present invention also provides a method of alleviating a decrease in muscle mass and muscle function, including administering a food composition including *Lactobacillus gasseri* BNR17 with accession number KCTC 10902BP or a culture thereof. Furthermore, the present invention provides the use of *Lactobacillus gasseri* BNR17 with accession number KCTC 10902BP or a culture thereof for the manufacture of a food composition for alleviating a decrease in muscle mass and muscle function.

The present invention provides a composition for improving muscular strength and muscular endurance, including *Lactobacillus gasseri* BNR17 with accession number KCTC 10902BP or a culture thereof. The present invention also provides a method of improving muscular strength and muscular endurance, including administering a composition including *Lactobacillus gasseri* BNR17 with accession number KCTC 10902BP or a culture thereof. Furthermore, the present invention provides the use of *Lactobacillus gasseri* BNR17 with accession number KCTC 10902BP or a culture thereof for the manufacture of a composition for improving muscular strength and muscular endurance.

The present invention provides an anticancer adjuvant including *Lactobacillus gasseri* BNR17 with accession number KCTC 10902BP or a culture thereof.

### [Description of Drawings]

FIGs. 1 to 4 show results of comparison of inhibitory effects of various Lactobacillus sp. microorganisms on sarcopenia caused by glucocorticoid as a stress hormone;
FIGs. 5 to 10 show results of comparison of inhibitory effects of various *Lactobacillus gasseri* strains on sarcopenia caused by the stress hormone glucocorticoid;
FIGs. 11 to 22 show effects and mechanisms of *Lactobacillus gasseri* BNR17 on sarcopenia caused by the stress hormone glucocorticoid;
FIGs. 23 to 31 show effects of *Lactobacillus gasseri* BNR17 on sarcopenia caused by cisplatin as a drug used in anticancer chemotherapy;
FIGs. 32 to 39 show effects of *Lactobacillus gasseri* BNR17 on sarcopenia caused by a tumor;
FIGs. 40 to 43 show effects of *Lactobacillus gasseri* BNR17 on sarcopenia caused by obesity; and
FIGs. 44 to 46 show effects of *Lactobacillus gasseri* BNR17 on sarcopenia caused by aging.

### [Mode for Invention]

Unless otherwise defined, all technical and scientific terms used herein have the same meanings as typically understood by those skilled in the art to which the present invention belongs. In general, the nomenclature used herein and the experimental methods described below are well known in the art and are typical.

In a specific embodiment according to the present invention, it has been confirmed that *Lactobacillus gasseri* BNR17 with accession number KCTC 10902BP or a culture thereof is capable of preventing or treating sarcopenia, alleviating a decrease in muscle mass and muscle function, and improving muscular strength and muscular endurance, and is usable as an anticancer adjuvant.

An aspect of the present invention relates to a composition for preventing or treating sarcopenia, including *Lactobacillus gasseri* BNR17 with accession number KCTC 10902BP or a culture thereof.

Another aspect of the present invention relates to a food composition for alleviating a decrease in muscle mass and muscle function, including *Lactobacillus gasseri* BNR17 with accession number KCTC 10902BP or a culture thereof.

Still another aspect of the present invention relates to a composition for improving muscular strength and muscular endurance, including *Lactobacillus gasseri* BNR17 with accession number KCTC 10902BP or a culture thereof.

Yet another aspect of the present invention relates to an anticancer adjuvant including *Lactobacillus gasseri* BNR17 with accession number KCTC 10902BP or a culture thereof.

The strain according to the present invention has been deposited at the Korea Research Institute of Bioscience and Biotechnology on February 01, 2006 under KCTC 10902BP.

The *Lactobacillus gasseri* BNR17 with accession number KCTC 10902BP or the culture thereof may exhibit:
inhibition of a decrease in MyoD (myoblast determination protein-1);
inhibition of a decrease in MyHC (myosin heavy chain) ;
inhibition of an increase in MuRF1 (muscle RING-finger protein-1);
inhibition of an increase in MAFbx (muscle atrophy F-box, also known as atrogin-1);
inhibition of an increase in FOXO3 (forkhead box O3); and
inhibition of an increase in GDF-15 (growth differentiation factor 15).

In relation to the culture of the strain, "culture" may mean a result of culturing in a culture medium or liquid culture containing the strain. The culture may or may not contain the strain. The culture may be provided in the form of a liquid or a solid, but the present invention is not limited thereto.

Various forms of the culture may be included, such as a concentrate, dried product, or extract of the culture.

The extract may be obtained by extraction using water, an organic solvent, etc., for example, water, a lower alcohol having 1 to 4 carbon atoms, hexane, chloroform, ethyl acetate, or a mixed solvent thereof.

"Prevention" means any action that inhibits or delays the onset of the disease by administering the composition according to the present invention. "Treatment or alleviation" means any action that ameliorates or beneficially changes the symptoms of a disease.

The decrease in muscular strength and muscular endurance may be caused by at least one factor selected from the group consisting of sarcopenia, muscle loss, muscle weakness, atony, muscular atrophy, and myasthenia gravis.

More specifically, the decrease in muscular strength and muscular endurance may be caused by at least one factor selected from the group consisting of sarcopenia, muscle loss, muscle weakness, atony, muscular atrophy, myasthenia gravis, spastic spine syndrome, amyotrophic lateral sclerosis, cachexia, Charcot-Marie-Tooth disease, muscle degradation, muscle degeneration, and muscular dystrophy.

In the present invention, sarcopenia is a decrease in muscle mass in the body and is a disease that is characterized by a decrease in muscle tissue due to a disease of the muscle itself or a decrease in muscle tissue due to damage to the nerves that control the muscle.

Sarcopenia may be caused by a tumor or drug treatment, and specifically, the drug may be an anticancer drug. Tumor or drug treatment may cause a decrease in muscle mass and function such as muscle reduction, muscle loss, a decrease in muscular strength, a decrease in muscular endurance, etc., and the present invention has uses for the prevention, alleviation, or treatment of sarcopenia or a decrease in muscle mass and muscle function in that the prevention, alleviation, and treatment effects on such a decrease in muscle function were confirmed in the examples.

The composition may further alleviate any symptom selected from the group consisting of increased fatigue, weight loss, and decreased appetite.

The composition for preventing or treating sarcopenia according to the present invention may be used alone or in combination with other pharmaceutically active compounds, as well as in an appropriate formulations therewith.

The composition of the present invention may additionally include a pharmaceutically acceptable carrier.

The composition of the present invention may be prepared into a pharmaceutical formulation using methods well known in the art to provide rapid, sustained, or delayed release of the active ingredient after administration to a mammal. In preparing the formulation, it is preferable to mix or dilute the active ingredient with a carrier or encapsulate the same in a carrier in the form of a container.

Therefore, the composition of the present invention may be formulated and used in the form of oral formulations such as powders, granules, tablets, capsules, suspensions, emulsions, syrups, aerosols, etc., external preparations, suppositories, and sterile injectable solutions according to typical methods, and may further include appropriate carriers, excipients, and diluents commonly used in the preparation of the composition.

Examples of the carrier that may be included in the composition of the present invention include, but are not limited to, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. Formulations are prepared using commonly used diluents or excipients such as fillers, extenders, binders, wetting agents, disintegrants, surfactants, etc.

Solid formulations for oral administration include tablets, pills, powders, granules, capsules, etc. These solid formulations are obtained by mixing the compound with at least one excipient, for example, starch, calcium carbonate, sucrose or lactose, gelatin, etc. In addition to simple excipients, lubricants such as magnesium stearate and talc are also used.

Liquid formulations for oral administration include suspensions, oral solutions, emulsions, syrups, etc. In addition to the commonly used simple diluents such as water and liquid paraffin, various excipients, for example, wetting agents, sweeteners, fragrances, preservatives, and the like, may be included.

Formulations for parenteral administration include sterile aqueous solutions, non-aqueous vehicles, suspensions, emulsions, lyophilized preparations, and suppositories. Non-aqueous vehicles and suspensions may include propylene glycol, polyethylene glycol, plant oil such as olive oil, injectable ester such as ethyl oleate, and the like. As a base for suppositories, Witepsol, Macrogol, Tween 61, cacao butter, laurin oil, glycerogelatin, etc. may be used.

The concentration of the active ingredient included in the composition may be determined taking into consideration the purpose of treatment, patient condition, required period, etc., and is not limited to a specific range. The pharmaceutical composition of the present invention is administered in a pharmaceutically effective amount. In the present invention, "pharmaceutically effective amount" is an amount sufficient to treat a disease at a reasonable benefit/risk ratio applicable to any medical treatment, and the effective amount may be determined depending on factors including the type and severity of the patient's disease, drug activity, drug sensitivity, time of administration, route of administration and excretion rate, duration of treatment, and concurrently used drugs, and other factors well known in the field of medicine.

The pharmaceutical composition of the present invention may be administered as an individual therapeutic agent or in combination with other therapeutic agents, and may be administered sequentially or simultaneously with conventional therapeutic agents. Also, the composition may be administered once or multiple times. Taking into consideration all of the above factors, it is important to administer an amount that may achieve maximum effect in a minimum amount without causing side effects, which may be easily determined by those skilled in the art.

As used herein, the term "administration" means introducing the pharmaceutical composition of the present invention into a patient by any appropriate method, and the composition of the present invention may be administered through various routes such as oral or parenteral routes, so long as it may reach the target tissue.

The mode of administration of the pharmaceutical composition according to the present invention is not particularly limited, and may follow a method commonly used in the art. As a non-limiting example of the mode of administration, the composition may be administered orally or parenterally. The pharmaceutical composition according to the present invention may be prepared in various dosage forms depending on the desired administration mode.

The frequency of administration of the composition of the present invention is not particularly limited, but the composition may be administered once a day or several times a day in divided doses.

A typical daily dose of the composition according to the present invention may be 2.5×10⁴ to 2.5×10¹² cfu/day, particularly 2.5×10⁶ to 2.5×10¹⁰ cfu/day, which may be administered once or several times.

As used herein, the term "subject" may refer to any animal, including a human, that has developed or is likely to develop a disease. The animal may be a mammal, such as not only a human but also a cow, a horse, a sheep, a pig, a goat, a camel, an antelope, a dog, a cat, and the like that require treatment for similar symptoms, but is not limited thereto.

The prevention or treatment method of the present invention may specifically include administering the composition in a pharmaceutically effective amount to a subject that has developed or is at risk of developing the disease.

The food composition may be for improving exercise performance ability.

The food composition of the present invention may be consumed on a daily basis, and is thus very useful as it may be expected to have a prevention or alleviation effect on the target disease.

As used herein, the term "alleviation" refers to any action in which a disease is ameliorated or beneficially changed by administration of the composition of the present invention.

The food composition of the present invention is provided in forms such as pills, powders, granules, precipitates, tablets, capsules, or liquids. Examples of the food to which the composition of the present invention may be added include various foods, such as beverages, gums, teas, vitamin complexes, health supplements, and the like.

There is no particular limitation on other ingredients in addition to containing the composition having the activity of preventing and treating the target disease including the active material, or the active ingredient thereof, or the physiologically acceptable salt thereof, as an essential ingredient that may be included in the food composition of the present invention. Like typical foods, various herbal extracts, food supplements, or natural carbohydrates may be contained as additional ingredients.

The food may be a functional health food. Here, "functional health food" refers to a food with high medical effects processed to efficiently exhibit bioregulatory functions in addition to supplying nutrients. A functional health food may be manufactured in various forms such as tablets, capsules, powders, granules, liquid, pills, etc. to obtain useful effects in preventing or alleviating a disease.

A functional health food composition may be manufactured into foods, particularly functional foods. The functional food of the present invention may include ingredients commonly added during food production, examples of which may include proteins, carbohydrates, fats, nutrients, and seasonings. For example, when manufactured as a drink, natural carbohydrates or flavoring agents may be included as additional ingredients in addition to the active ingredient. The natural carbohydrates preferably include monosaccharides (e.g., glucose, fructose, etc.), disaccharides (e.g., maltose, sucrose, etc.), oligosaccharides, polysaccharides (e.g., dextrin, cyclodextrin, etc.), or sugar alcohols (e.g., xylitol, sorbitol, erythritol, etc.). The flavoring agent may be a natural flavoring agent (e.g., thaumatin, stevia extract, etc.) or a synthetic flavoring agent (e.g., saccharin, aspartame, etc.).

There is no particular limitation on types of functional health food. Examples of the functional health food may include dairy products, beverages, tea drinks, alcoholic beverages, and vitamin complexes, and all health foods in the typical sense may be included.

In addition, various nutrients, vitamins, electrolytes, flavors, colorants, pectic acid and salts thereof, alginic acid and salts thereof, organic acids, protective colloidal thickeners, pH adjusters, stabilizers, preservatives, glycerin, alcohol, carbonating agents used in carbonated beverages, etc. may be further included.

The present invention relates to an anticancer adjuvant including *Lactobacillus gasseri* BNR17 with accession number KCTC 10902BP or a culture thereof. Since it has been confirmed that *Lactobacillus gasseri* BNR17 is effective at preventing or treating sarcopenia caused by tumors or drug treatment using anticancer drugs, etc., the anticancer drug and the anticancer adjuvant may be administered simultaneously or sequentially.

The anticancer drug may be at least one selected from the group consisting of cisplatin, doxorubicin, irinotecan, paclitaxel, daunorubicin, docetaxel, 5-fluorouracil and the like.

As used herein, the term "anticancer adjuvant" refers to an adjuvant that has the effect of alleviating the side effects of anticancer drugs during anticancer treatment. The side effects of the anticancer drugs may include sarcopenia, a decrease in muscle mass and muscle function, a decrease in muscular strength and muscular endurance, fatigue, weight loss, decreased appetite, and the like.

In addition to including *Lactobacillus gasseri* BNR17 as an active ingredient, the anticancer adjuvant may further include at least one active ingredient that exhibits the same or similar functions. The anticancer adjuvant may be administered orally or parenterally during clinical administration, and upon parenteral administration, intraperitoneal injection, intrarectal injection, subcutaneous injection, intravenous injection, intramuscular injection, intrauterine dural injection, intracerebroventricular injection, or intrathoracic injection is possible. The anticancer adjuvant may be used in the form of general pharmaceutical preparations.

The anticancer adjuvant may be used alone or in combination with surgery, radiotherapy, hormone therapy, chemotherapy, and methods using biologicals and the like.

The daily dose of the anticancer adjuvant may be 2.5×10⁴ to 2.5×10¹² cfu/day, particularly 2.5×10⁶ to 2.5×10¹⁰ cfu/day, which is preferably administered once a day or several times a day, but the range thereof varies depending on the patient's body weight, age, sex, health condition, diet, administration time, administration method, excretion rate, and severity of the disease. The anticancer adjuvant of the present invention may be administered in various parenteral formulations during actual clinical administration. When formulated, commonly used diluents or excipients such as fillers, extenders, binders, wetting agents, disintegrants, surfactants, etc. are used. Formulations for parenteral administration may include sterilized aqueous solutions, non-aqueous vehicles, suspensions, emulsions, lyophilized preparations, and suppositories. Non-aqueous vehicles and suspensions may include propylene glycol, polyethylene glycol, plant oil such as olive oil, injectable ester such as ethyl oleate, and the like. As a base for suppositories, Witepsol, Macrogol, Tween 61, cacao butter, laurin oil, glycerogelatin, etc. may be used.

Still yet another aspect of the present invention relates to a method of inhibiting a decrease in muscle mass and muscle function accompanying sarcopenia using *Lactobacillus gasseri* BNR17: a method of inhibiting a decrease in muscular strength and muscular endurance; a method of inhibiting a decrease in myosin and myoblast determination protein 1 (MyoD) and a method of inhibiting an increase in growth differentiation factor-15 (GDF-15).

Myosin controls muscle contraction through interaction with actin, and myoblast determination protein 1 (MyoD) is a transcription factor that plays a key role in increasing muscle mass, and expression thereof is activated by exercise (Rudnicki et al., Cell 75(7): 1351-1359, 1993). The decrease in proteins that control muscle function and muscle mass is triggered by stress, obesity, aging, medication, tumorigenesis, etc., and is the most direct factor that causes sarcopenia.

Muscle RING-finger 1 (MuRF1) and muscle atrophy F-box (MAFbx) are muscle-specific ubiquitin E3 ligases, which play a key role in ubiquitin-dependent proteolysis of actin-myosin and MyoD (Polge et al., FASEB J 25:3790-3802, 2011; Lokireddy et al., Am J Physiol Cell Physiol 303:C512-C529, 2012). Moreover, the expression of MuRF1 and MAFbx in muscle is achieved by the forkhead box O3 (FOXO3) transcription factor (Bodine et al., Am J Physiol Endocrinol Metab 307:E469-E484, 2014).

Growth differentiation factor-15 (GDF-15) is known to be an indicator of tissue damage that increases when various tissues including the lungs, kidneys, and liver are damaged, and damage to various tissues in the human body causes a decrease in muscle function (Zimmers et al., Shock 23:543-548, 2005). In particular, since GDF-15 is associated with sarcopenia caused by tumors, it has been reported that sarcopenia caused by tumors may be treated when inhibiting GDF-15 using methods such as monoclonal antibodies and the like (Wu et al., Annals of Oncology 31: S245-S259, 2020).

A better understanding of the present invention may be obtained through the following examples. These examples are merely set forth to illustrate the present invention, and are not to be construed as limiting the scope of the present invention, as will be apparent to those of ordinary skill in the art.

### Example 1: Comparative experiment of inhibitory effects of various Lactobacilli on sarcopenia caused by stress hormone

Lactobacillus sp. such as *Lactobacillus casei* (LC: accession number KCTC 3109^{T}), *Lactobacillus paragasseri* (LP: accession number KCTC 3510^{T}), *Lactobacillus ruteri* (LR: accession number KCTC 3594^{T}), and *Lactobacillus gasseri* (LG: accession number KCTC 10902BP) were isolated from fermented foods, human feces, human milk, etc. and cultured in MRS medium for 16 hours.

The experimental animals were 4-week-old female Balb/c mice (18 to 20 g), supplied by Central Lab. Animal Inc., and were acclimatized for one week in a laboratory animal facility and then used in the experiment. During the experiment period, solid feed and water were freely consumed, and the temperature (22±2°C), relative humidity (60±5%), and 12-hour light/dark cycle of the breeding room were maintained.

The mice were divided into control and glucocorticoid (stress hormone) treatment groups, and the glucocorticoid treatment groups were further divided into a vehicle administration group and Lactobacillus administration groups. The control group was intraperitoneally injected with 200 µl of saline, and the glucocorticoid treatment groups were intraperitoneally injected with 30 mg/kg of glucocorticoid once daily.

At the same time, among the glucocorticoid treatment groups, the vehicle administration group was administered distilled water, and the Lactobacillus administration groups were orally administered various Lactobacilli such as LC, LP, LR, LG, and LRG (1:1 mixture of LR and LG) at doses of 10⁸ cfu/mouse (2.5×10¹⁰ cfu/human when converted into human dose) for 3 weeks (2.5×10¹⁰ cfu/human = 10⁸ cfu/20 g x 60,000 g/12, mouse standard body weight: 20 g, human standard body weight: 60 kg, human/mouse dose equivalent factor: 12).

After administration, muscular endurance of the mice was measured using a treadmill (Columbus Instrument, USA). Muscular endurance was measured by the maximum exercise time and maximum exercise distance until the mouse was exhausted and could no longer run. The lean body mass of each mouse was measured by dual energy X-ray absorptiometry using an InAlyzer (Medikors, Korea). After termination of the experiment, the mice were sacrificed and the weights of gastrocnemius and quadriceps were measured.

### Example 2: Comparison of inhibitory effects of various Lactobacilli on sarcopenia caused by stress hormone

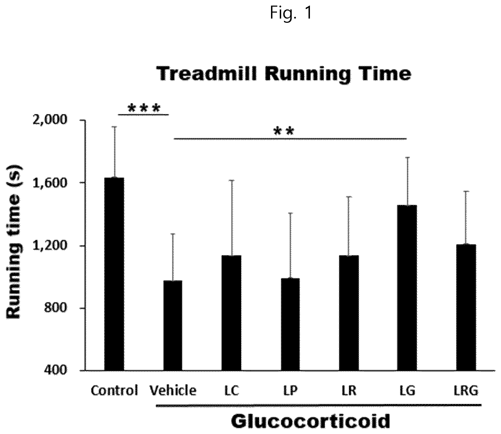

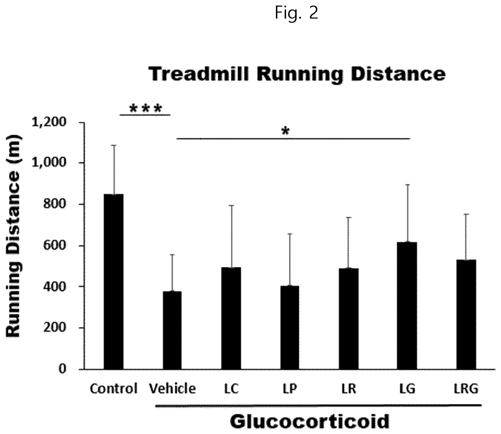

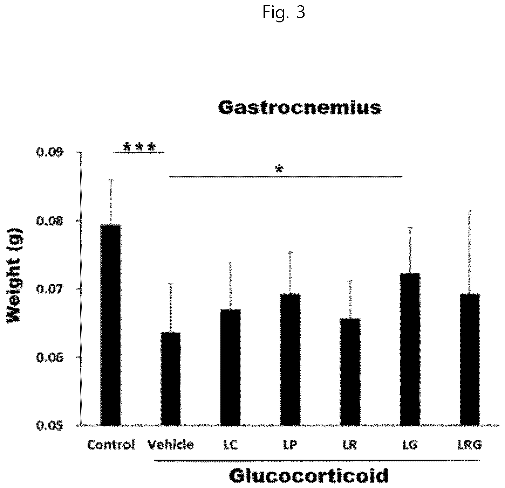

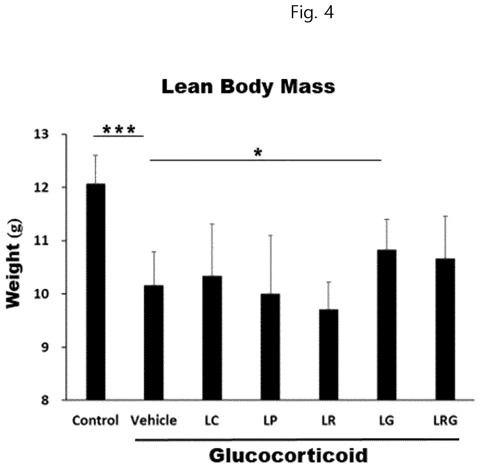

Based on results of measurement of muscular endurance of the mice using a treadmill, both the treadmill running time and distance were very low in the vehicle administration group of the glucocorticoid groups compared to the control group. Muscular endurance was significantly recovered in the *Lactobacillus gasseri* (LG) administration group among the groups administered various Lactobacilli (FIGs. 1 and 2). In addition, the weight of gastrocnemius was significantly increased in the *Lactobacillus gasseri* administration group (FIG. 3). The lean body mass measured by dual energy X-ray absorptiometry was significantly decreased in the vehicle administration group of the glucocorticoid groups compared to the control group, but the lean body mass was significantly increased in the *Lactobacillus gasseri* administration group (FIG. 4).

Among various Lactobacillus sp., *Lactobacillus gasseri* exhibited a significant sarcopenia inhibitory effect, so subsequent experiments were performed using *Lactobacillus gasseri.*

### Example 3: Comparative experiment of inhibitory effects of various Lactobacillus gasseri strains on sarcopenia caused by stress hormone

Various *Lactobacillus gasseri* strains, namely H65 *(Lactobacillus gasseri* H65: accession number KCCM 80404), H30 *(Lactobacillus gasseri* H30: accession number KCCM 80403), RM2 *(Lactobacillus gasseri* RM2, accession number KCCM 80405), FF557 *(Lactobacillus gasseri* FF557, accession number KCCM 80406), and BNR17 were cultured in MRS medium for 16 hours.

The experimental animals were 4-week-old female Balb/c mice (18 to 20 g), supplied by Central Lab. Animal Inc., and were acclimatized for one week in a laboratory animal facility and then used in the experiment. During the experiment period, solid feed and water were freely consumed, and the temperature (22±2°C), relative humidity (60±5%), and 12-hour light/dark cycle of the breeding room were maintained.

The mice were divided into control and glucocorticoid (stress hormone) treatment groups, and the glucocorticoid treatment groups were further divided into a vehicle administration group and Lactobacillus administration groups. The control group was intraperitoneally injected with 200 µl of saline, and the glucocorticoid treatment groups were intraperitoneally injected with 30 mg/kg of glucocorticoid once daily.

At the same time, among the glucocorticoid treatment groups, the vehicle administration group was administered distilled water, and the Lactobacillus administration groups were orally administered various *Lactobacillus gasseri* strains such as H65, H30, RM2, FF557, and BNR17 at doses of 10⁸ cfu/mouse (2.5×10¹⁰ cfu/human when converted into human dose) for 16 days (2.5×10¹⁰ cfu/human = 10⁸ cfu/20 g x 60,000 g/12, mouse standard body weight: 20 g, human standard body weight: 60 kg, human/mouse dose equivalent factor: 12).

Body weight change during the administration period and final body weight were measured. Muscular endurance of the mice was measured using a treadmill (Columbus Instrument, USA). Muscular endurance was quantified by the maximum exercise time and maximum exercise distance until the mouse was exhausted and could no longer run. Forelimb muscular strength of the mice was measured using a grip strength meter (Bioseb, France). After termination of the experiment, the mice were sacrificed and the weights of gastrocnemius and quadriceps were measured.

### Example 4: Comparison of inhibitory effects of various Lactobacillus gasseri strains on sarcopenia caused by stress hormone

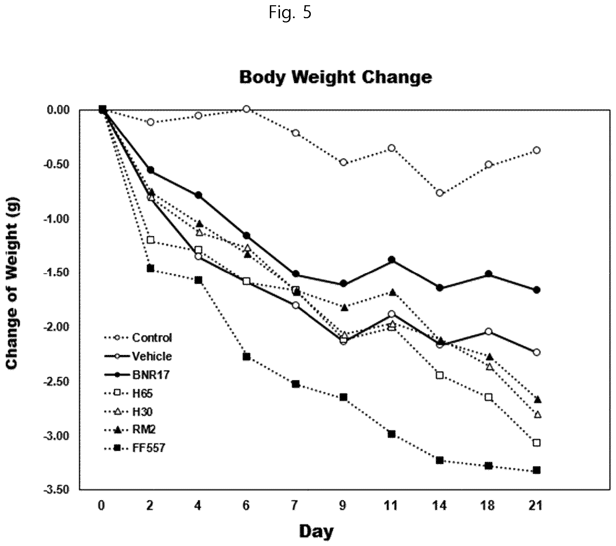

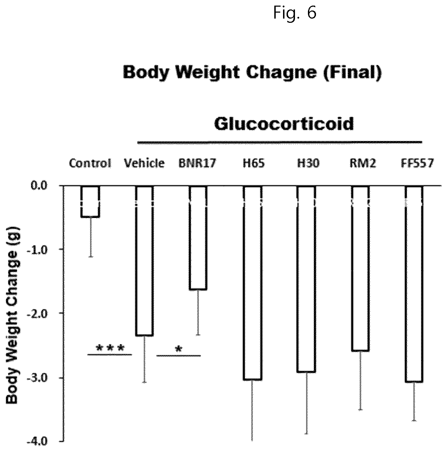

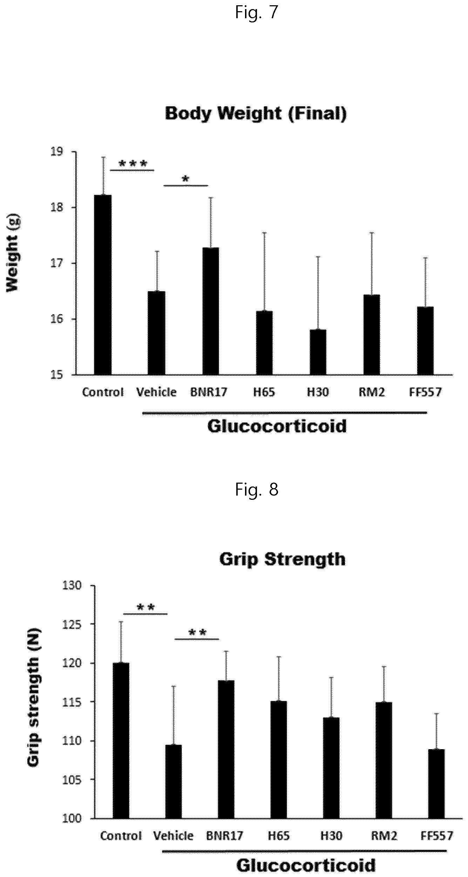

The body weight of mice was reduced by the administration of glucocorticoid. Only the group administered BNR17 among various *Lactobacillus gasseri* strains exhibited less body weight loss in the glucocorticoid treatment groups, and the other strains showed similar or more severe body weight loss (FIG. 5). Final body weight was significantly decreased in the glucocorticoid treatment groups compared to the control group, and glucocorticoid-induced body weight loss was significantly inhibited only in the BNR17 administration group (FIG. 6). The weight of quadriceps was significantly decreased in the glucocorticoid treatment groups compared to the control group, and the glucocorticoid-induced weight loss of quadriceps was significantly inhibited only in the BNR17 administration group (FIG. 7). Based on results of measurement of muscular strength (grip strength) of the mice using a grip strength meter, the grip strength was significantly decreased in the glucocorticoid treatment groups compared to the control group, and the decrease in grip strength caused by glucocorticoid was significantly inhibited only in the BNR17 administration group (FIG. 8).

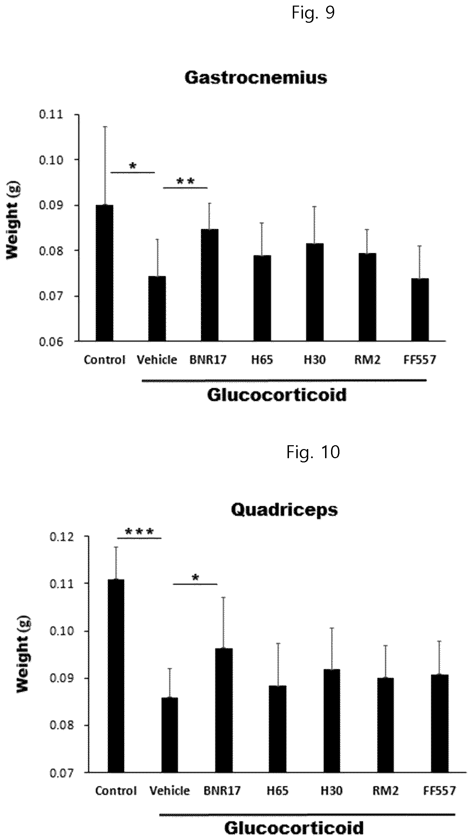

Based on results of measurement of muscular endurance of the mice using a treadmill, both the treadmill running time and distance were very low in the vehicle administration group of the glucocorticoid groups compared to the control group. Muscular endurance was significantly recovered in the group administered BNR17 among various *Lactobacillus gasseri* strains (FIGs. 9 and 10).

Among various *Lactobacillus gasseri* strains, *Lactobacillus gasseri* BNR17 exhibited a significant sarcopenia inhibitory effect, so subsequent experiments were performed using *Lactobacillus gasseri* BNR17.

### Example 5: Experiment of inhibitory efficacy and mechanism on sarcopenia caused by stress hormone

*A Lactobacillus gasseri* BNR17 strain was cultured in MRS medium for 16 hours.

The experimental animals were 4-week-old female Balb/c mice (18 to 20 g), supplied by Central Lab. Animal Inc., and were acclimatized for one week in a laboratory animal facility and then used in the experiment. During the experiment period, solid feed and water were freely consumed, and the temperature (22±2°C), relative humidity (60±5%), and 12-hour light/dark cycle of the breeding room were maintained.

The mice were divided into control and glucocorticoid (stress hormone) groups, and the glucocorticoid groups were further divided into a vehicle administration group and *Lactobacillus gasseri* administration groups. The control group was intraperitoneally injected with 200 µl of saline, and the glucocorticoid groups were intraperitoneally injected with 30 mg/kg of glucocorticoid once daily.

At the same time, among the glucocorticoid groups, the vehicle administration group was administered distilled water, and the *Lactobacillus gasseri* administration groups were orally administered *Lactobacillus gasseri* at doses of 10⁷ to 10⁸ cfu/mouse (2.5×10⁹ to 2.5×10¹⁰ cfu/human when converted into human dose) for 14 days.

After administration, muscular endurance of the mice was measured using a treadmill (Columbus Instrument, USA). Muscular endurance was quantified by the maximum exercise time and maximum exercise distance until the mouse was exhausted and could no longer run. Also, forelimb muscular strength of the mice was measured using a grip strength meter (Bioseb, France). The lean body mass of the whole body and hind calf of each mouse was measured using an InAlyzer (Medikors, Korea) by dual energy X-ray absorptiometry.

After termination of the experiment, the mice were sacrificed and the weights of quadriceps and gastrocnemius were measured. Proteins were extracted from the quadriceps of each mouse and the levels of myosin heavy chain (MyHC), myoblast determination protein 1 (MyoD), MuRF1, MAFbx, FOXO3, and beta-actin (control protein) were measured by Western blotting, and quantitative analysis was performed compared to beta-actin (control protein) using ImageJ software (NIH, Bethesda, MD, USA).

### Example 6: Confirmation of inhibitory effect and mechanism of Lactobacillus gasseri BNR17 on sarcopenia caused by stress hormone

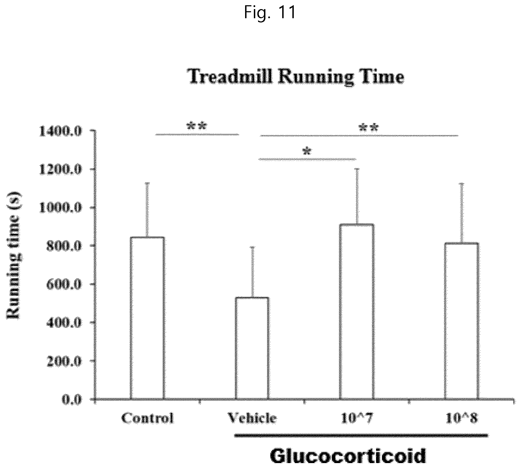

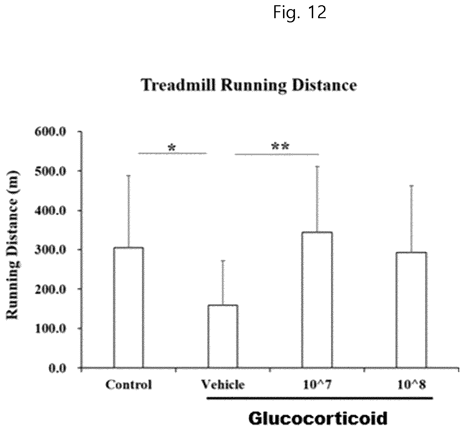

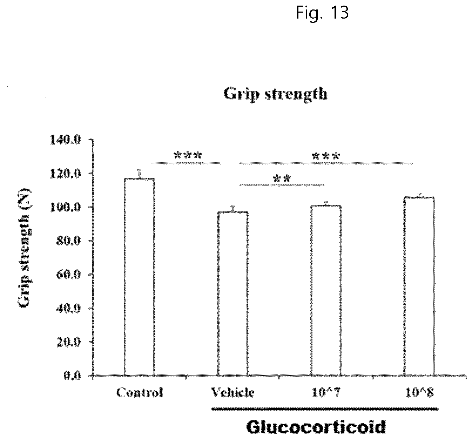

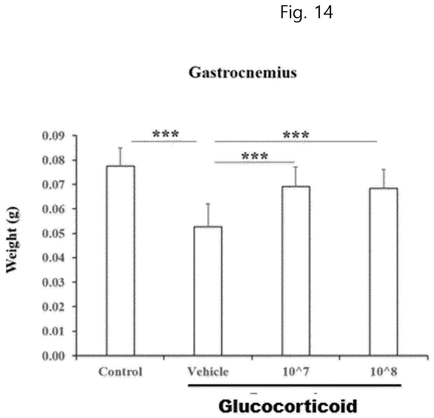

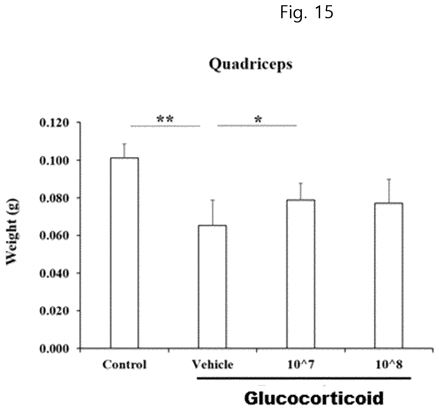

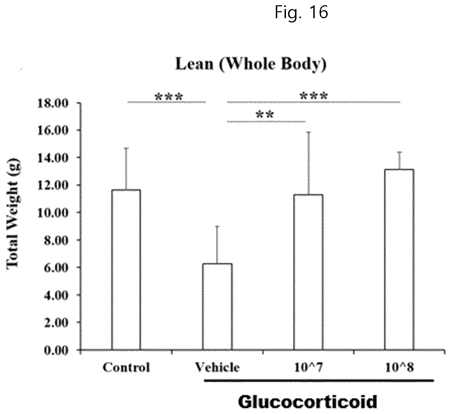

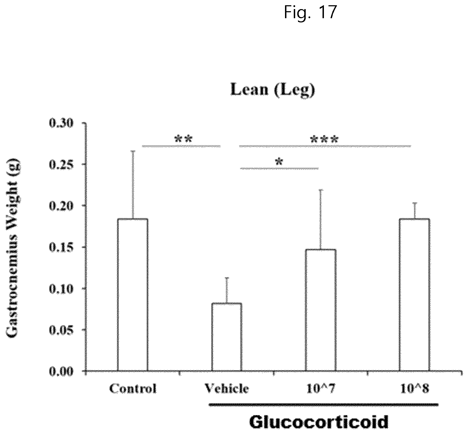

Based on results of measurement of muscular endurance of the mice using a treadmill, both the treadmill running time and distance were very low in the vehicle administration group of the glucocorticoid (stress hormone) groups compared to the control group. However, muscular endurance was significantly recovered in the *Lactobacillus gasseri* BNR17 administration groups (FIGs. 11 and 12). Muscular strength (grip strength) of the mice was significantly decreased in the vehicle administration group of the glucocorticoid groups compared to the control group, but the grip strength was significantly recovered in the *Lactobacillus gasseri* BNR17 administration groups (FIG. 13). The weights of gastrocnemius and quadriceps were significantly increased in the *Lactobacillus gasseri* BNR17 administration groups (FIGs. 14 and 15). The lean body mass of the whole body and the lean body mass of the hind legs measured by dual energy X-ray absorptiometry were significantly decreased in the vehicle administration group of the glucocorticoid groups compared to the control group, but the lean body mass of the whole body and the lean body mass of the hind legs were significantly increased in the *Lactobacillus gasseri* BNR17 administration groups (FIGs. 16 and 17).

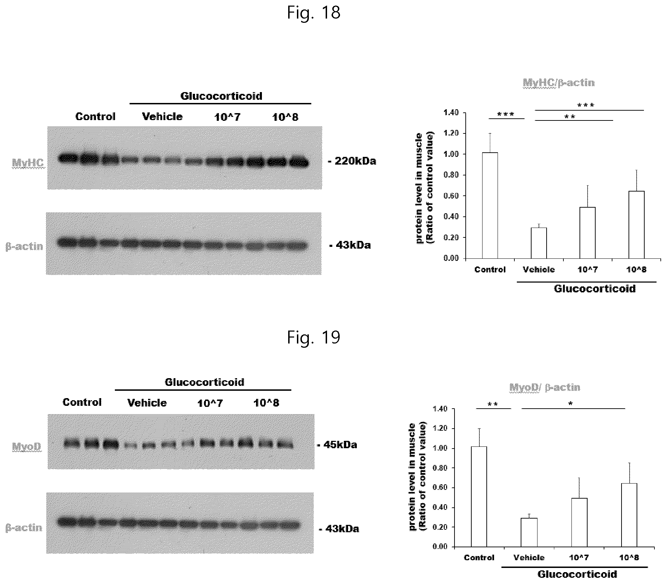

Muscle contraction occurs through the interaction of myosin and actin. Based on results of measurement of the level of myosin heavy chain (MyHC), which plays a key role in muscle contraction, by protein extraction from the quadriceps muscle, the level of MyHC was decreased in the vehicle administration group of the glucocorticoid groups compared to the control group, but the level of MyHC was significantly increased in the *Lactobacillus gasseri* BNR17 administration groups compared to the vehicle administration group of the glucocorticoid groups (FIG. 18).

Myoblast determination protein 1 (MyoD) is a transcription factor that plays a key role in increasing muscle mass, and expression thereof is activated by exercise (Rudnicki et al., Cell 75(7): 1351-1359, 1993). Based on results of measurement of the level of MyoD by protein extraction from the quadriceps muscle, the level of MyoD was decreased in the vehicle administration group of the glucocorticoid groups compared to the control group, but the level of MyoD was significantly increased in the *Lactobacillus gasseri* BNR17 administration groups compared to the vehicle administration group of the glucocorticoid groups (FIG. 19).

The increased levels of MyHC, which plays a key role in muscle contraction, and MyoD, which plays a key role in increasing muscle mass, suggest a direct mechanism of action of *Lactobacillus gasseri* BNR17 on the increase in muscular endurance, muscular strength, and muscle mass.

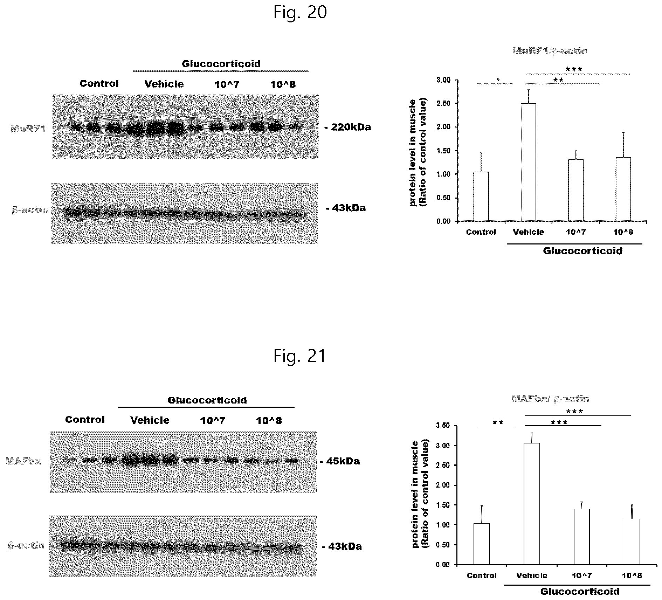

The levels of MuRF1 and MAFbx, which cause ubiquitin-dependent proteolysis of MyHC and MyoD, were increased in the vehicle administration group of the glucocorticoid groups compared to the control group, but these levels were significantly decreased in the *Lactobacillus gasseri* BNR17 administration groups compared to the vehicle administration group of the glucocorticoid groups (FIGs. 20 and 21).

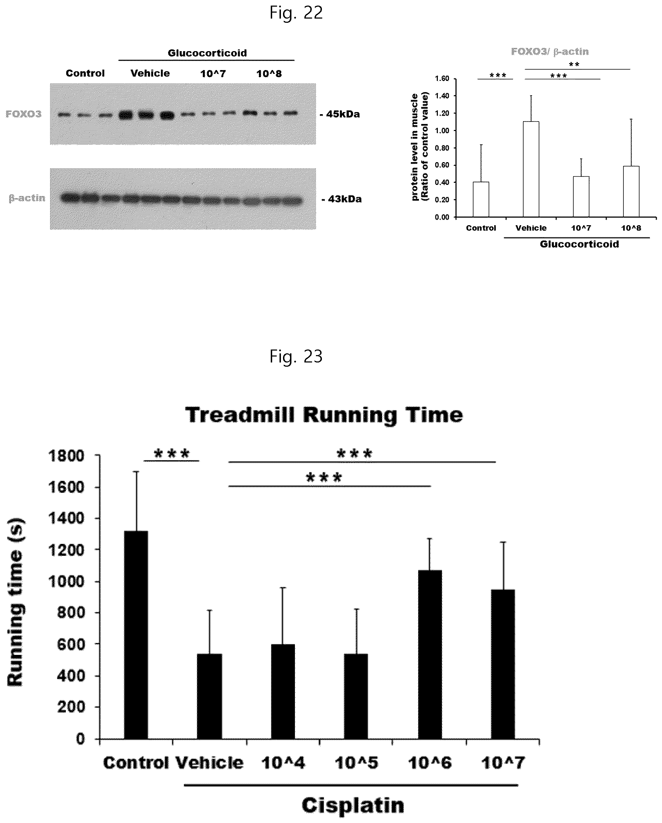

The level of FOXO3, a transcription factor that causes transcription of MuRF1 and MAFbx, was also increased in the vehicle administration group of the glucocorticoid groups compared to the control group, but the level of FOXO3 was significantly decreased in the *Lactobacillus gasseri* BNR17 administration groups compared to the vehicle administration group of the glucocorticoid groups (FIG. 22).

### Example 7: Experiment of inhibitory effect on sarcopenia caused by drug

The experimental animals were 4-week-old female Balb/c mice (18 to 20 g), supplied by Central Lab. Animal Inc., and were acclimatized for one week in a laboratory animal facility and then used in the experiment. During the experiment period, solid feed and water were freely consumed, and the temperature (22±2°C), relative humidity (60±5%), and 12-hour light/dark cycle of the breeding room were maintained.

Cisplatin was used to prepare drug-induced sarcopenia model mice. Drugs used in anticancer chemotherapy, including cisplatin, cause sarcopenia as a major side effect, which reduces cure rates and quality of life (Davis MP et al., Annals of Palliative Medicine, 8(1): 86-101, 2019)

The mice were divided into control and cisplatin groups, and the cisplatin groups were further divided into a vehicle administration group and *Lactobacillus gasseri* administration groups. The control group was intraperitoneally injected with 200 µl of saline, and the cisplatin groups were intraperitoneally injected with 3 mg/kg of cisplatin four times at two-day intervals.

At the same time, among the cisplatin groups, the vehicle administration group was administered distilled water, and the *Lactobacillus gasseri* administration groups were orally administered *Lactobacillus gasseri* at doses of 10⁴ to 10⁷ cfu/mouse (2.5×10⁶ to 2.5×10⁹ cfu/human when converted into human dose) for 9 days.

After administration, muscular endurance of the mice was measured using a treadmill (Columbus Instrument, USA). Muscular endurance was quantified by the maximum exercise time and maximum exercise distance until the mouse was exhausted and could no longer run. Also, forelimb muscular strength of the mice was measured using a grip strength meter (Bioseb, France).

After termination of the experiment, the mice were sacrificed, blood was collected from the heart, and serum was separated by centrifugation. Proteins were extracted from the quadriceps of each mouse, and the levels of myosin heavy chain (MyHC), myoblast determination protein 1 (MyoD), and beta-actin (control protein) were measured by Western blotting, and quantitative analysis was performed compared to beta-actin (control protein) using ImageJ software (NIH, Bethesda, MD, USA). GDF-15 in muscle and serum was quantified by ELISA.

### Example 8: Confirmation of effect of Lactobacillus gasseri BNR17 on sarcopenia caused by drug

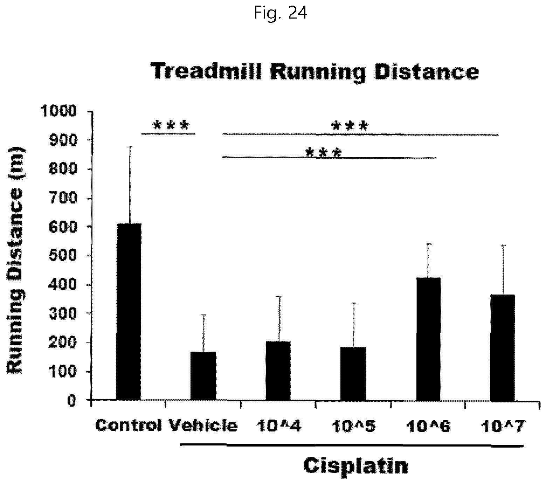

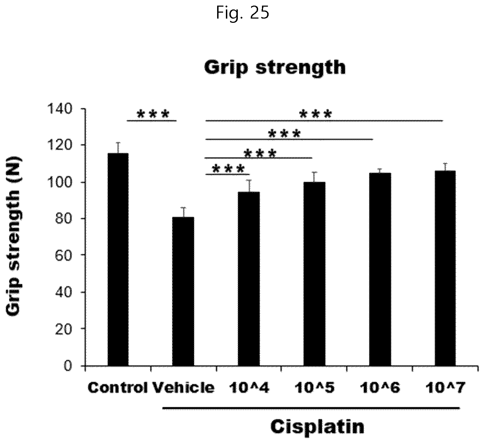

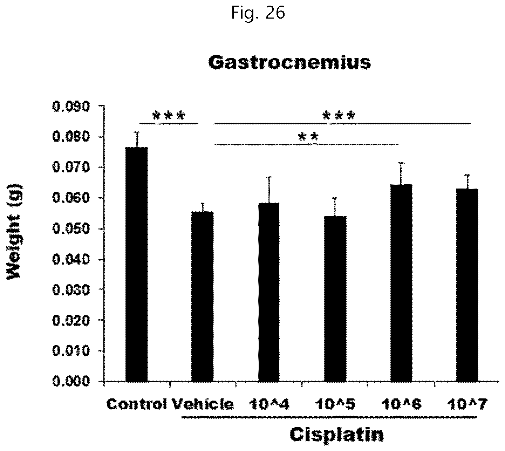

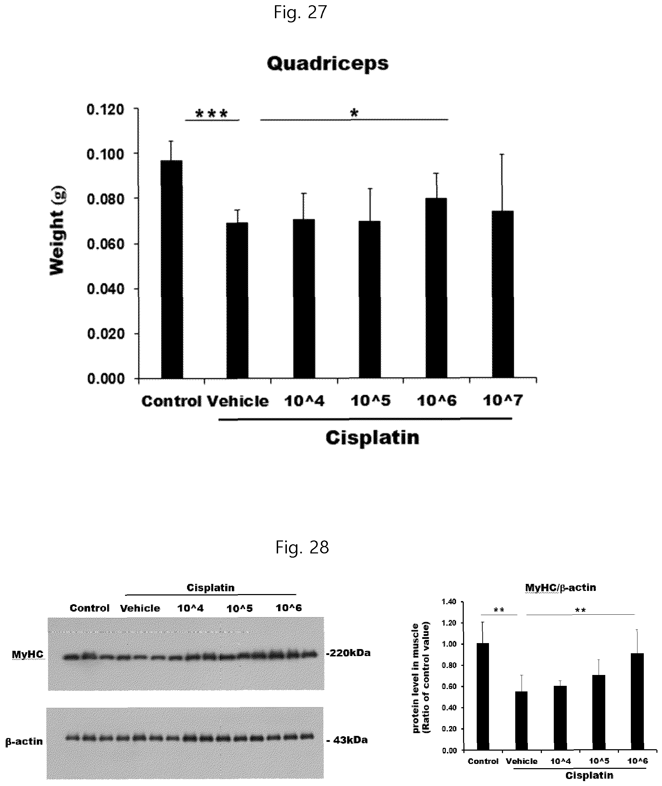

Based on results of measurement of muscular endurance of the mice using a treadmill, the treadmill running time and distance were significantly decreased in the vehicle administration group of the cisplatin groups compared to the control group. However, an excellent muscular endurance recovery effect was confirmed in the *Lactobacillus gasseri* BNR17 administration groups (FIGs. 23 and 24). Muscular strength (grip strength) of the mice was significantly decreased in the vehicle administration group of the cisplatin groups compared to the control group, but the grip strength was significantly recovered in the *Lactobacillus gasseri* BNR17 administration groups (FIG. 25). The weights of gastrocnemius and quadriceps were significantly increased in the *Lactobacillus gasseri* BNR17 administration groups (FIGs. 26 and 27).

Muscle contraction occurs through the interaction of myosin and actin. Based on results of measurement of the level of MyHC (myosin heavy chain), which plays a key role in muscle contraction, by protein extraction from the quadriceps muscle, the level of MyHC was decreased in the vehicle administration group of the cisplatin groups compared to the control group, but the level of MyHC was significantly increased in the *Lactobacillus gasseri* BNR17 administration groups compared to the vehicle administration group of the cisplatin groups (FIG. 28).

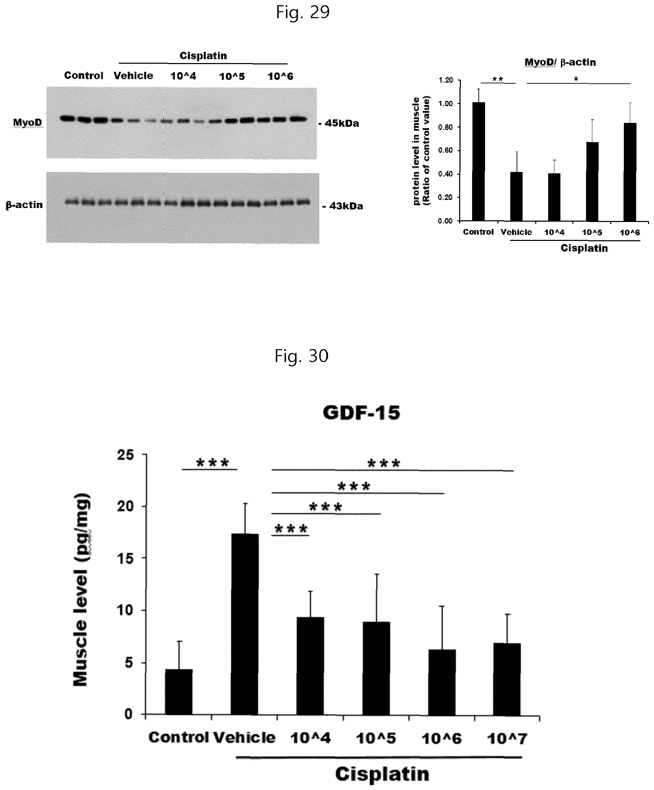

Myoblast determination protein 1 (MyoD) is a transcription factor that plays a key role in increasing muscle mass, and expression thereof is activated by exercise (Rudnicki et al., Cell 75(7): 1351-1359, 1993). Based on results of measurement of the level of MyoD by protein extraction from the quadriceps muscle, the level of MyoD was decreased in the vehicle administration group of the cisplatin groups compared to the control group, but the level of MyoD was significantly increased in the *Lactobacillus gasseri* BNR17 administration groups compared to the vehicle administration group of the cisplatin groups (FIG. 29).

The increased levels of MyHC, which plays a key role in muscle contraction, and MyoD, which plays a key role in increasing muscle mass, suggest a direct mechanism of action of *Lactobacillus gasseri* on the increase in muscular endurance, muscular strength, and muscle weight.

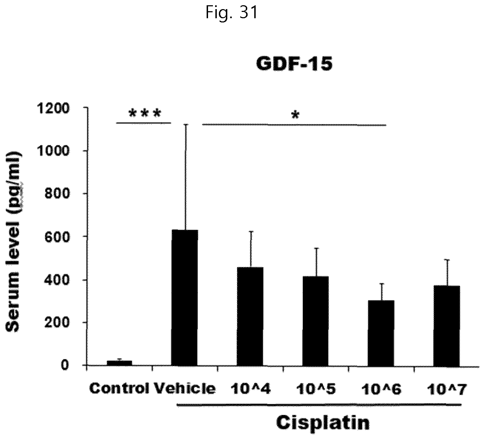

Based on results of quantification of GDF-15 in muscle, the level of GDF-15 in muscle was greatly increased in the vehicle administration group of the cisplatin groups compared to the control group, but the level of GDF-15 in muscle was significantly decreased in the *Lactobacillus gasseri* administration groups (FIG. 30). The results of quantification of GDF-15 in serum showed the same pattern as the level of GDF-15 in muscle (FIG. 31).

### Example 9: Experiment of inhibitory effect on sarcopenia caused by tumor

The experimental animals were 4-week-old female Balb/c mice (18 to 20 g), supplied by Central Lab. Animal Inc., and were acclimatized for one week in a laboratory animal facility and then used in the experiment. During the experiment period, solid feed and water were freely consumed, and the temperature (22±2°C), relative humidity (60±5%), and 12-hour light/dark cycle of the breeding room were maintained.

The mice were divided into control and tumor groups, and the tumor groups were further divided into a vehicle administration group and *Lactobacillus gasseri* administration groups. The control group was subcutaneously injected with 200 µl of saline, and the tumor groups were subcutaneously injected with 10⁶ cells/mouse of C26 colon carcinoma cells.

After 14 days had elapsed and the tumor had clearly formed, the vehicle administration group was administered distilled water, and the comparative druggroup was orally administered 160 mg/kg of megestrol acetate (MA), which is the most commonly used medicine to treat body weight loss in cancer patients, once daily for 7 days (mouse dose calculation: 160 mg/kg = 800 mg/60 kg x 12, daily human dose: 800 mg, human standard body weight: 60 kg, human/mouse dose equivalent factor: 12).

The *Lactobacillus gasseri* administration groups were orally administered *Lactobacillus gasseri* BNR17 at doses of 10⁵ to 10⁷ cfu/mouse for 7 days.

After administration, muscular endurance of the mice was measured using a treadmill (Columbus Instrument, USA), and muscular strength of the mice was measured using a grip strength meter (Bioseb, France).

After termination of the experiment, the mice were sacrificed, blood was collected from the heart, and serum was separated by centrifugation. Proteins were extracted from the quadriceps of each mouse, and the levels of myosin heavy chain (MyHC), myoblast determination protein 1 (MyoD), and beta-actin (control protein) were measured by Western blotting, and quantitative analysis was performed compared to beta-actin (control protein) using ImageJ software (NIH, Bethesda, MD, USA). GDF-15 in muscle and serum was quantified by ELISA.

### Example 10: Confirmation of effect of Lactobacillus gasseri BNR17 on sarcopenia caused by tumor

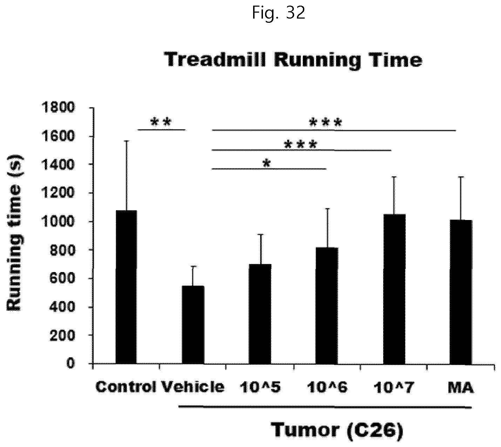

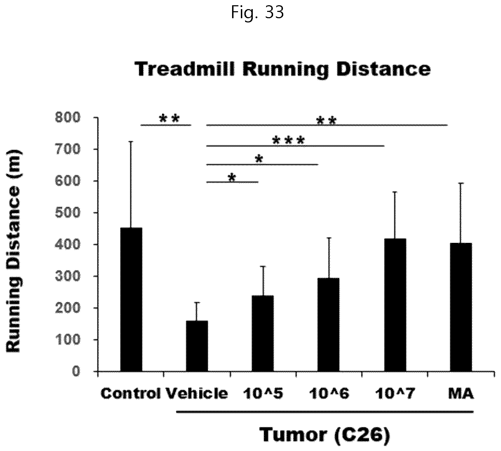

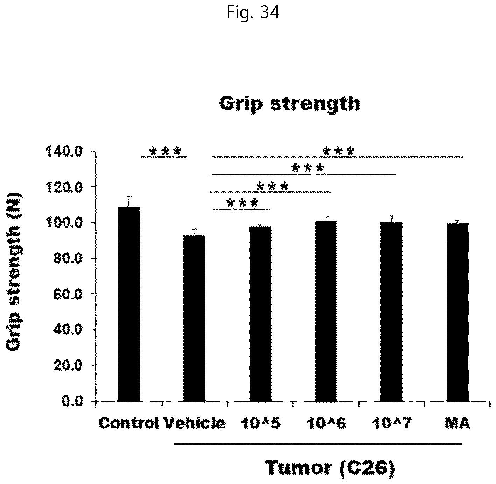

Based on results of measurement of muscular endurance of the mice using a treadmill, both the treadmill running time and distance were very low in the vehicle administration group of the tumor groups compared to the control group. However, muscular endurance was significantly increased in the *Lactobacillus gasseri* BNR17 administration groups (FIGs. 32 and 33). Muscular strength (grip strength) of the mice was significantly decreased in the vehicle administration group of the tumor groups compared to the control group, but the grip strength was significantly recovered in the *Lactobacillus gasseri* BNR17 administration groups (FIG. 34).

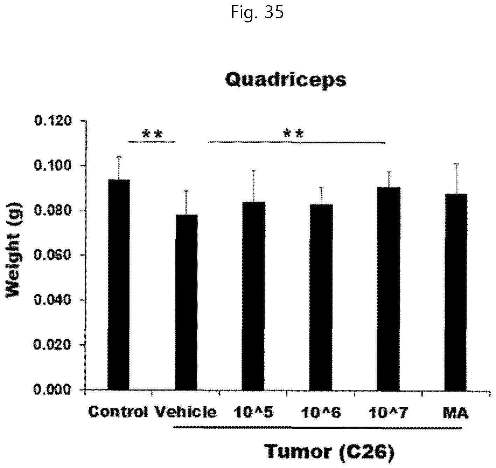

Also, based on results of measurement and comparison of the weight of quadriceps, the weight of quadriceps was significantly decreased in the vehicle administration group of the tumor groups compared to the control group, but the muscle weight was significantly increased in the *Lactobacillus gasseri* BNR17 administration groups (FIG. 35).

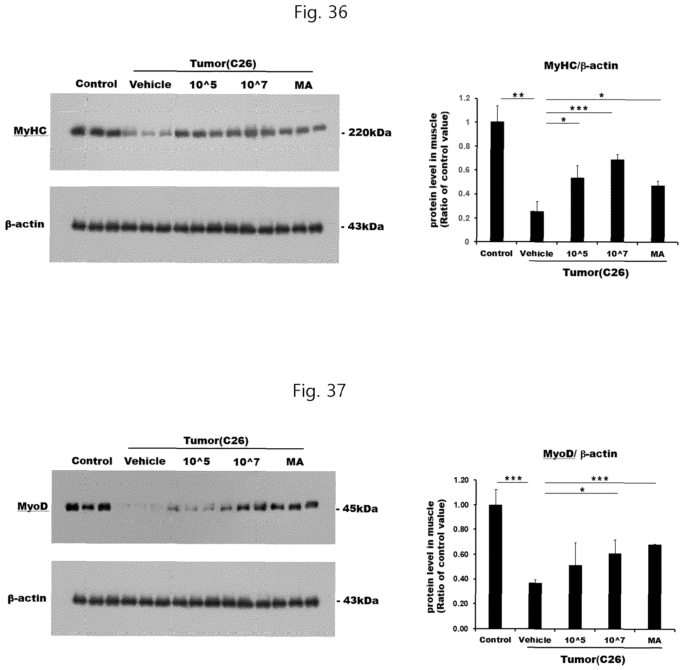

Based on results of measurement of the level of MyHC (myosin heavy chain), which plays a key role in muscle contraction, by protein extraction from the quadriceps muscle, the level of MyHC was decreased in the vehicle administration group of the tumor groups compared to the control group, but the level of MyHC was significantly increased in the *Lactobacillus gasseri* BNR17 administration groups compared to the vehicle administration group of the tumor groups (FIG. 36).

Based on results of measurement of the level of MyoD, which plays a key role in increasing muscle mass, by protein extraction from the quadriceps muscle, the level of MyoD was decreased in the vehicle administration group of the tumor groups compared to the control group, but the level of MyoD was significantly increased in the *Lactobacillus gasseri* BNR17 administration groups compared to the vehicle administration group of the tumor groups (FIG. 37).

The increased levels of MyHC, which plays a key role in muscle contraction, and MyoD, which plays a key role in increasing muscle mass, suggest a direct mechanism of action of *Lactobacillus gasseri* on the increase in muscular endurance, muscular strength, and muscle weight.

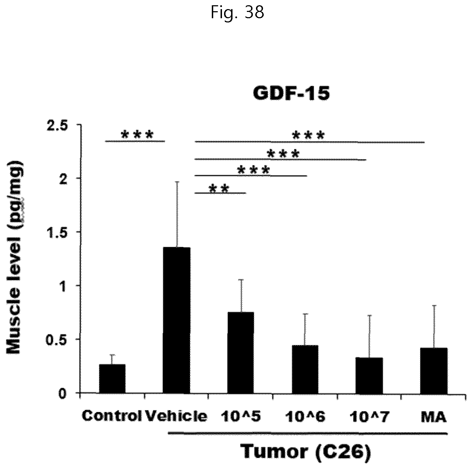

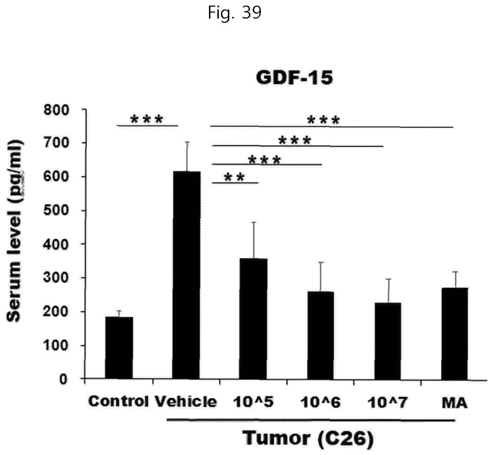

Based on results of quantification of GDF-15 in muscle, the level of GDF-15 in muscle was greatly increased in the vehicle administration group of the tumor groups compared to the control group, but the level of GDF-15 in muscle was significantly decreased in the *Lactobacillus gasseri* BNR17 administration groups (FIG. 38). The results of quantification of GDF-15 in serum showed the same pattern as the level of GDF-15 in muscle (FIG. 39).

In inhibiting sarcopenia caused by a tumor, *Lactobacillus gasseri* BNR17 at 10⁷ cfu/mouse (2.5×10⁹ cfu/human) exhibited the same or superior efficacy compared to megestrol acetate (MA), the most commonly used medicine to treat body weight loss in cancer patients.

### Example 11: Experiment of inhibitory effect on sarcopenia caused by obesity

The experimental animals were 4-week-old female Balb/c mice (18 to 20 g), supplied by Central Lab. Animal Inc., and were acclimatized for one week in a laboratory animal facility and then used in the experiment. During the experiment period, solid feed and water were freely consumed, and the temperature (22±2°C), relative humidity (60±5%), and 12-hour light/dark cycle of the breeding room were maintained.

The mice were divided into control and obese groups, and the obese groups were further divided into a vehicle administration group and *Lactobacillus gasseri* BNR17 administration groups. The control group was fed regular feed, and the obese groups were fed a high fat diet (70% fat) for 8 weeks to induce obesity.

Thereafter, among the obese groups, the vehicle administration group was administered distilled water, and the *Lactobacillus gasseri* administration groups were orally administered *Lactobacillus gasseri* BNR17 at doses of 10⁷ to 10⁸ cfu/mouse (2.5×10⁹ to 2.5×10¹⁰ cfu/human when converted into human dose) for 8 weeks.

After administration, muscular endurance of the mice was measured using a treadmill (Columbus Instrument, USA). Muscular endurance was quantified by the maximum exercise time and maximum exercise distance until the mouse was exhausted and could no longer run. Also, forelimb muscular strength of the mice was measured using a grip strength meter (Bioseb, France). The lean body mass of each mouse was measured using an InAlyzer (Medikors, Korea) by dual energy X-ray absorptiometry.

### Example 12: Confirmation of inhibitory effect of Lactobacillus gasseri BNR17 on sarcopenia caused by obesity

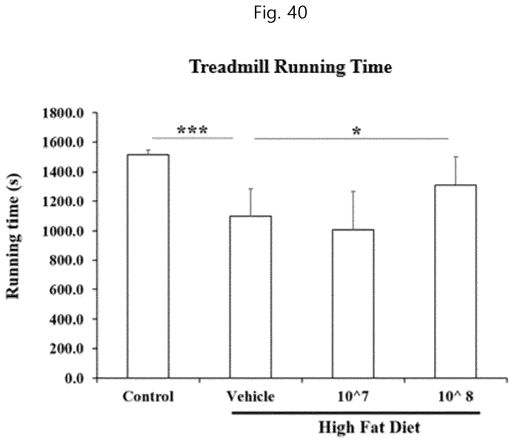

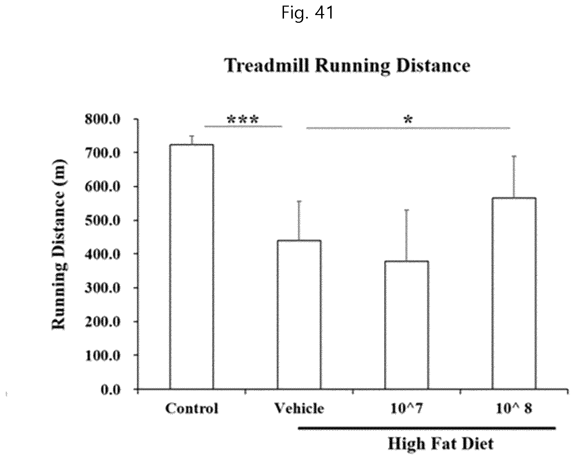

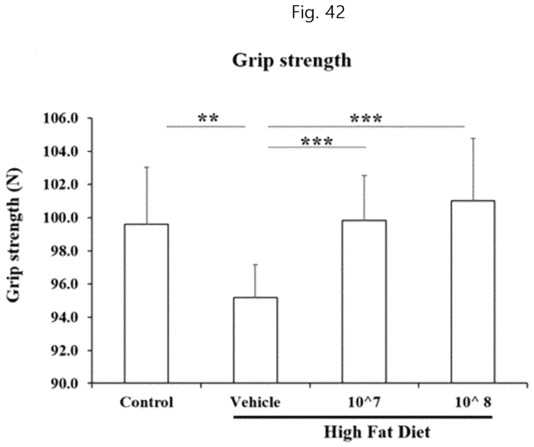

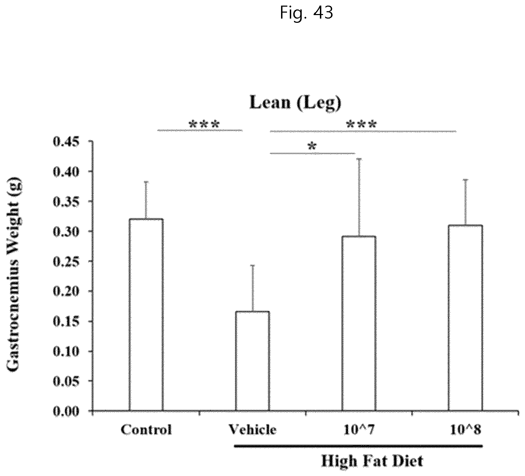

Based on results of measurement of muscular endurance of the mice using a treadmill, both the treadmill running time and distance were very low in the vehicle administration group of the obese groups compared to the control group. However, muscular endurance was significantly recovered in the *Lactobacillus gasseri* BNR17 administration group (FIGs. 40 and 41). Muscular strength (grip strength) of the mice was significantly decreased in the vehicle administration group of the obese groups compared to the control group, but the grip strength was significantly recovered in the *Lactobacillus gasseri* BNR17 administration groups (FIG. 42). The lean body mass of the hind legs measured by dual energy X-ray absorptiometry was significantly decreased in the vehicle administration group of the obese groups compared to the control group, but the lean body mass of the hind legs was significantly increased in the *Lactobacillus gasseri* BNR17 administration groups (FIG. 43).

### Example 13: Experiment of inhibitory effect on sarcopenia caused by aging

The experimental animals were 4-week-old female Balb/c mice (18 to 20 g), supplied by Central Lab. Animal Inc., and were acclimatized for one week in a laboratory animal facility and then used in the experiment. During the experiment period, solid feed and water were freely consumed, and the temperature (22±2°C), relative humidity (60±5%), and 12-hour light/dark cycle of the breeding room were maintained.

The mice were divided into control and aging induction groups, and the aging induction groups were further divided into a vehicle administration group and *Lactobacillus gasseri* BNR17 administration groups. The control group was intraperitoneally injected with 200 µl of saline, and the aging induction groups were intraperitoneally injected with 200 mg/kg of D-galactose for 6 weeks to induce aging (Chang L et al., Journal of Medicinal Food 17(3): 357-364, 2014).

At the same time, among the aging induction groups, the vehicle administration group was administered distilled water, and the D-galactose administration groups were orally administered *Lactobacillus gasseri* BNR17 at doses of 10⁶ to 10⁸ cfu/mouse (2.5×10⁸ to 2.5×10¹⁰ cfu/human when converted into human dose).

After administration, muscular endurance of the mice was measured using a treadmill (Columbus Instrument, USA). Muscular endurance was quantified by the maximum exercise time and maximum exercise distance until the mouse was exhausted and could no longer run. Also, forelimb muscular strength of the mice was measured using a grip strength meter (Bioseb, France).

### Example 14: Confirmation of inhibitory effect of Lactobacillus gasseri BNR17 on sarcopenia caused by aging

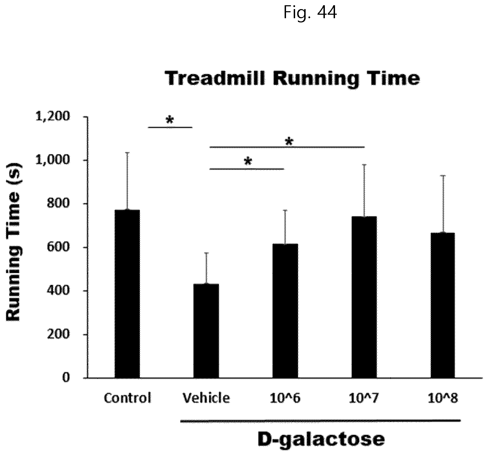

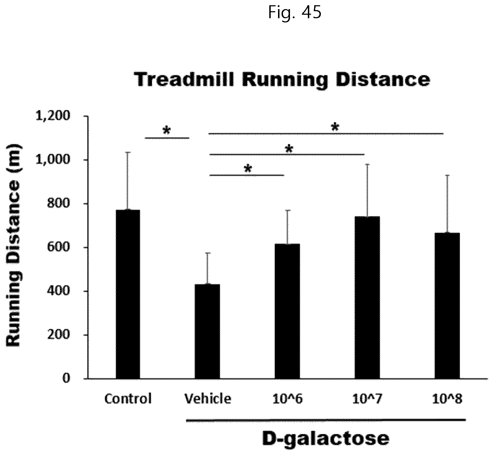

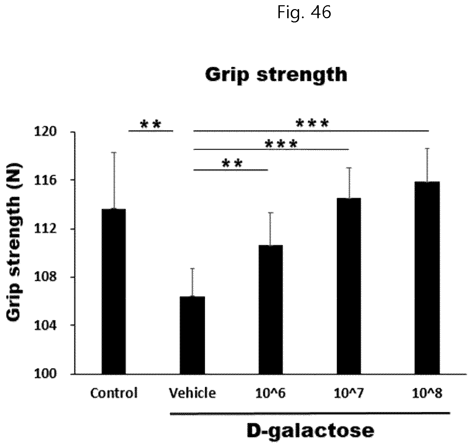

Based on results of measurement of muscular endurance of the mice using a treadmill, both the treadmill running time and distance were very low in the vehicle administration group of the aging induction groups compared to the control group. However, muscular endurance was significantly recovered in the *Lactobacillus gasseri* BNR17 administration groups (FIGs. 44 and 45). Muscular strength (grip strength) of the mice was significantly decreased in the vehicle administration group of the aging induction groups compared to the control group, but the grip strength was significantly recovered in the *Lactobacillus gasseri* BNR17 administration groups (FIG. 46).

In conclusion, the decrease in MyoD (myoblast determination protein-1), which plays a major role in increasing muscle mass, and MyHC (myosin heavy chain), which plays a major role in muscle contraction, acts as a direct factor that causes sarcopenia, which is deemed to be due to an increase in MuRF1 (muscle RING-finger protein-1) and MAFbx (muscle atrophy F-box, also known as atrogin-1), which cause ubiquitin-dependent proteolysis of MyoD and MyHC, and an increase in FOXO3 (forkhead box O3), which acts as a transcription factor of MuRF1 and MAFbx.

*Lactobacillus gasseri* BNR17 is capable of effectively suppressing a decrease in muscle function and muscle mass by inhibiting the decrease in MyHC and MyoD and the increase in MuRF1, MAFbx, and FOXO3 caused by various factors such as stress, drugs, tumors, obesity, aging, etc. Therefore, *Lactobacillus gasseri* BNR17 is usable as a pharmaceutical composition for preventing and treating sarcopenia caused by various factors including stress, drugs, tumors, obesity, and aging, and as a food for improving muscle mass and muscle function or muscular strength and muscular endurance.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### [Industrial Applicability]

According to embodiments of the present invention, a pharmaceutical composition and a food composition can prevent or treat sarcopenia and improve muscle mass and muscle function or muscular strength and muscular endurance, thereby maintaining and improving health and quality of life in various situations such as stress, obesity, aging, medication, tumorigenesis, and the like.

The effects of the present invention are not limited to the foregoing, and other effects not mentioned herein will be clearly understood by those skilled in the art from this specification.

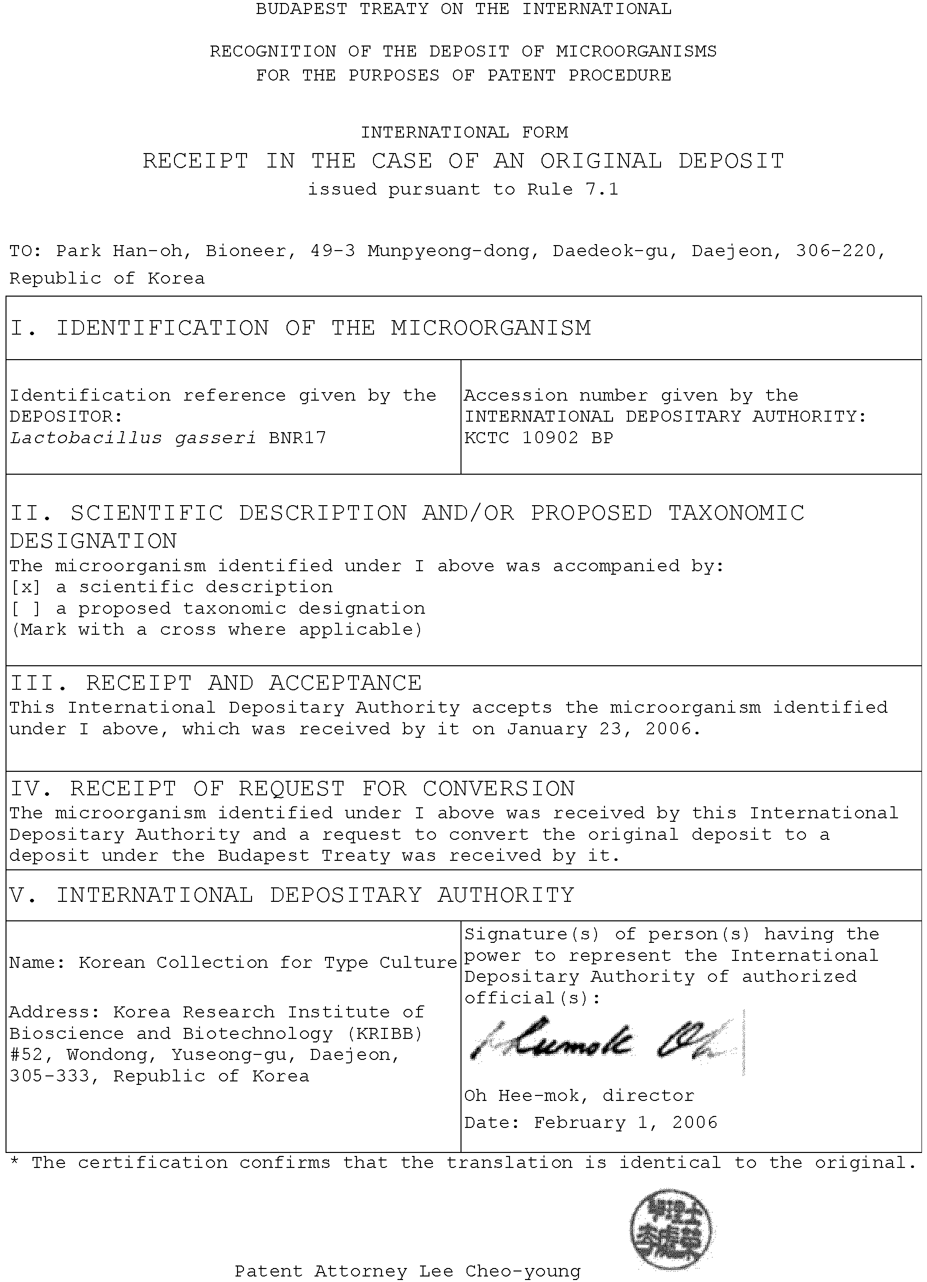

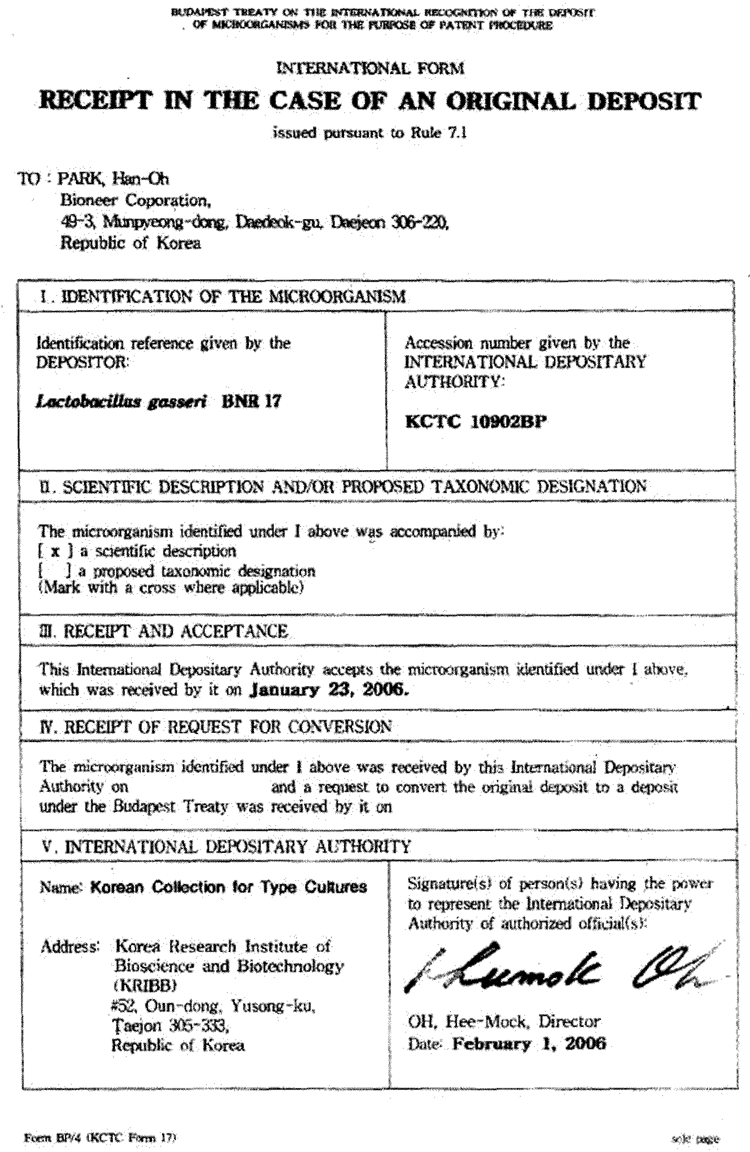

## Claims

1. A pharmaceutical composition for preventing or treating sarcopenia, comprising *Lactobacillus gasseri* BNR17 with accession number KCTC 10902BP or a culture thereof.

2. The pharmaceutical composition according to claim 1, wherein the sarcopenia is caused by stress, obesity, aging, a drug, or a tumor and the like.

3. The pharmaceutical composition according to claim 1, wherein the *Lactobacillus gasseri* BNR17 or the culture thereof exhibits:
inhibition of a decrease in MyoD (myoblast determination protein-1);
inhibition of a decrease in MyHC (myosin heavy chain);
inhibition of an increase in MuRF1 (muscle RING-finger protein-1);
inhibition of an increase in MAFbx (muscle atrophy F-box, atrogin-1);
inhibition of an increase in FOXO3 (forkhead box O3); and
inhibition of an increase in GDF-15 (growth differentiation factor 15).

4. A food composition for alleviating a decrease in muscle mass and muscle function, comprising *Lactobacillus gasseri* BNR17 with accession number KCTC 10902BP or a culture thereof.

5. The food composition according to claim 4, wherein the decrease in muscle mass and muscle function is caused by stress, obesity, aging, a drug, or a tumor and the like.

6. The food composition according to claim 4, wherein the *Lactobacillus gasseri* BNR17 or the culture thereof exhibits:
inhibition of a decrease in MyoD (myoblast determination protein-1);
inhibition of a decrease in MyHC (myosin heavy chain);
inhibition of an increase in MuRF1 (muscle RING-finger protein-1);
inhibition of an increase in MAFbx (muscle atrophy F-box, atrogin-1);
inhibition of an increase in FOXO3 (forkhead box O3); and
inhibition of an increase in GDF-15 (growth differentiation factor 15).

7. A composition for improving muscular strength and muscular endurance, comprising *Lactobacillus gasseri* BNR17 with accession number KCTC 10902BP or a culture thereof.

8. The composition according to claim 7, wherein the *Lactobacillus gasseri* BNR17 or the culture thereof exhibits:
inhibition of a decrease in MyoD (myoblast determination protein-1);
inhibition of a decrease in MyHC (myosin heavy chain);
inhibition of an increase in MuRF1 (muscle RING-finger protein-1);
inhibition of an increase in MAFbx (muscle atrophy F-box, atrogin-1);
inhibition of an increase in FOXO3 (forkhead box O3); and
inhibition of an increase in GDF-15 (growth differentiation factor 15).

9. An anticancer adjuvant comprising *Lactobacillus gasseri* BNR17 with accession number KCTC 10902BP or a culture thereof.

10. The anticancer adjuvant according to claim 9, for alleviating sarcopenia, a decrease in muscle mass and muscle function, or a decrease in muscular strength and muscular endurance, caused by an anticancer drug.
